(19) Europäisches Patentamt / European Patent Office / Office européen des brevets

(11) **EP 3 374 079 B1**

(12) **EUROPEAN PATENT SPECIFICATION**

(45) Date of publication and mention of the grant of the patent:
**12.03.2025 Bulletin 2025/11**

(21) Application number: **16793871.1**

(22) Date of filing: **08.11.2016**

(51) International Patent Classification (IPC):
**B01J 20/10** (2006.01)   **B01J 20/14** (2006.01)
**B01J 20/28** (2006.01)   **B01J 20/30** (2006.01)

(52) Cooperative Patent Classification (CPC):
**C01B 33/24; B01J 20/10; B01J 20/103; B01J 20/106; B01J 20/14; B01J 20/28011; B01J 20/28021; B01J 20/2803; B01J 20/3028; B01J 20/3042; C01B 33/18; C05G 5/12**

(86) International application number:
**PCT/EP2016/077024**

(87) International publication number:
**WO 2017/081031 (18.05.2017 Gazette 2017/20)**

(54) **HIGH ABSORPTION MINERALS**

HOCHABSORBIERENDE MINERALIEN

MINÉRAUX À HAUTE ABSORPTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.11.2015 EP 15306790**

(43) Date of publication of application:
**19.09.2018 Bulletin 2018/38**

(73) Proprietor: **IMERTECH SAS**
**75015 Paris (FR)**

(72) Inventors:
• **RILEY, Andrew Mark**
**St Columb Major**
**Cornwall TR9 6BQ (GB)**
• **PAGIS, Laure**
**31500 Toulouse (FR)**
• **ELTON-LEGRIX, Anabelle Huguette Renée**
**St. Austell**
**Cornwall PL26 7QL (GB)**
• **WANG, Bo**
**Union City**
**California 94587 (US)**

(74) Representative: **Haseltine Lake Kempner LLP**
**One Portwall Square**
**Portwall Lane**
**Bristol BS1 6BH (GB)**

(56) References cited:
**WO-A2-2011/101239    US-A1- 2005 005 870**
**US-A1- 2011 206 746    US-A1- 2014 371 061**

• WYPYCH ET AL: "Fillers - Origin, chemical composition, properties, and morphology", 1 January 2016 (2016-01-01), XP009535853, ISBN: 978-1-895198-91-1, Retrieved from the Internet <URL:https://www.sciencedirect.com/topics/engineering/fumed-silica> [retrieved on 20210909]
• ANONYMOUS: "ABOUT - Natural Sources", 1 January 2020 (2020-01-01), XP093126452, Retrieved from the Internet <URL:http://www.naturalsources.uk/about/> [retrieved on 20240201]

- ANONYMOUS: "Celtix(TM) Freshwater Diatomaceous Earth (Amorphous Silica, Fossil Shell Flour, Diatomite, Kieselguhr) - Ultra Fine Particle Size : Amazon.co.uk: Business, Industry & Science", 9 February 2018 (2018-02-09), XP093126495, Retrieved from the Internet <URL:https://www.amazon.co.uk/CeltixTM-Freshwater-Diatomaceous-Amorphous-Kieselguhr/dp/B079PWSNRH?th=1> [retrieved on 20240201]

**Description**

<u>TECHNICAL FIELD</u>

The present invention relates to uses of minerals and mineral composites having high acid and/or oil absorption capacities in personal care products.

<u>BACKGROUND OF THE INVENTION</u>

Mineral materials can be used for a wide range of applications in a wide range of products. One use of mineral materials is for absorption of substances such as water and/or organic compounds such as organic acids and oil in numerous applications. US 2005/005870 A1 discloses composite particles comprising a liquid absorbing material, which can be bentonite, zeolite, montmorillonite, diatomaceous earth, etc., and methods for making the same. The materials are useful as animal litter, carriers for actives in filters, in eliminating spills of toxic, organic, hazardous or non-toxic waste, in skin patches, as fertilizers, or in soaps, detergents, dry household products. It is therefore desirable to provide alternative and/or advantageous minerals and mineral compositions/composites. These alternative and/or advantageous minerals may each be suitable for use in particular applications.

<u>SUMMARY OF THE INVENTION</u>

In accordance with the present invention, there is provided the use of a silica- or silicate-based mineral in a personal care product according to claim 1.
According to the present invention, the mineral is diatomaceous earth.
Certain embodiments of any aspect of the present invention, may provide one or more of the following advantages:

- good (for example, high or increased) organic acid absorption properties;
- good (for example, high or increased) oil absorption properties;
- good (for example, high or increased) water absorption properties;
- good (for example, high or increased) odour absorption;
- good flowability (for example, even at relatively high oil and/or water absorption);
- reduced use of synthetic materials;
- improvement of (for example quick or quicker) drying time when used in liquid formulations;
- improvement of texture when used in liquid formulations and applied to skin.

The details, examples and preferences provided in relation to any particular one or more of the stated aspects of the present invention will be further described herein and apply equally to all aspects of the present invention.

<u>BRIEF DESCRIPTION OF THE FIGURES</u>

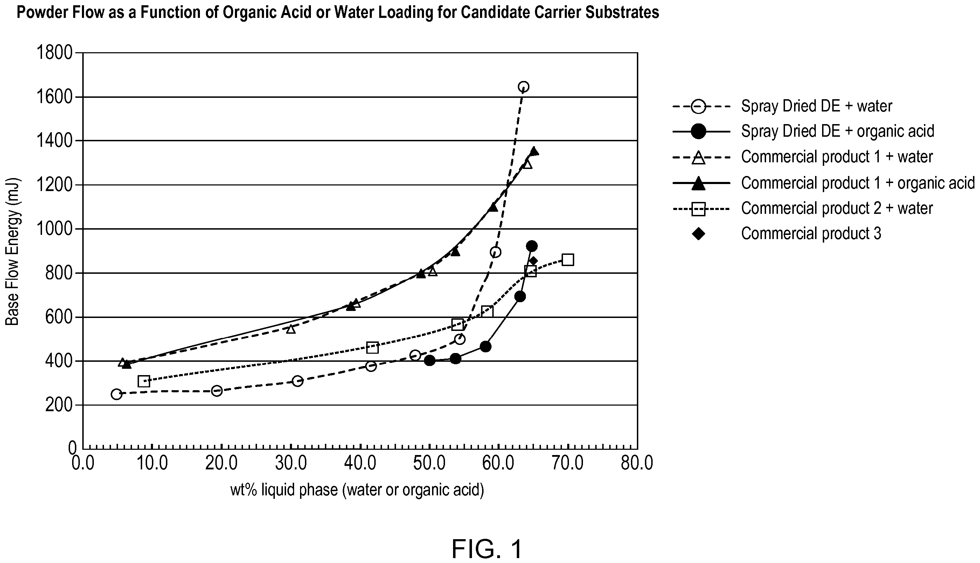

Fig. 1 shows the base flow energy of various minerals at different liquid (water or organic acid) loading levels.

<u>DETAILED DESCRIPTION OF THE INVENTION</u>

<u>Minerals</u>

There is provided herein minerals that are suitable for and/or intended for absorbing substances such as oil and/or water.
According to the present invention, the mineral is a silica-based mineral or a silicate-based mineral. A silica-based mineral is a mineral that comprises greater than 50 wt% silica ($SiO_2$). A silicate-based mineral is a mineral that comprises greater than 50 wt% of a chemical compound that comprises silicate ions (e.g. orthosilicate ions ($SiO_4^{4-}$) or other silicate ions ($[SiO_{2+n}]^{2n-}$)). For example, a silica-based mineral may be a mineral that comprises greater than 60 wt% or greater than 70 wt% or greater than 80 wt% or greater than 90 wt% silica. For example a silicate-based mineral may be a mineral that comprises greater than 60 wt% or greater than 70 wt% or greater than 80 wt% or greater than 90 wt% of a chemical compound that comprises silicate ions.
In certain embodiments, the mineral is naturally-derived (derived from a natural mineral). In certain embodiments, the mineral is synthetic. "Naturally-derived" means that the mineral is either naturally occurring or is made from a naturally occurring mineral. Any mineral that is naturally occurring or made from a naturally occurring mineral is not synthetic. Where the mineral is naturally-derived, it may be that some mineral impurities will inevitably contaminate the ground material. In general, however, the mineral will contain less than 5% by weight, preferably less than 1% by weight of other mineral impurities.

Silica-based minerals include both naturally-derived silica-based minerals and synthetic silica-based minerals. Naturally-derived silica-based minerals include, for example, free silica, natural glasses, diatomaceous earth or mixtures thereof. According to the present invention, the silica-based mineral is diatomaceous earth. Other silica-based materials are described herein below.

Free silica includes, for example, quartz, tridymite, cristobalite, opal, vitreous silica, coesite, stishovite and chalcedony.

Natural glasses (commonly referred to as volcanic glasses) are formed by the rapid cooling of siliceous magma or lava. Several types of natural glasses are known, including, for example, perlite, pumice, pumicite, shirasu, obsidian, and pitchstone. Prior to processing, perlite may be gray to green in color with abundant spherical cracks that cause it to break into small pearl-like masses. Pumice is a lightweight glassy vesicular rock. Obsidian may be dark in color with a vitreous luster and a characteristic conchoidal fracture. Pitchstone has a waxy resinous luster and may be brown, green, or gray. Volcanic glasses such as perlite and pumice occur in massive deposits and find wide commercial use. Volcanic ash, often referred to as "tuff" when in consolidated form, includes small particles or fragments that may be in glassy form.

As used herein, the term natural glass encompasses volcanic ash. Natural glasses may be chemically equivalent to rhyolite. Natural glasses that are chemically equivalent to trachyte, dacite, andesite, latite, and basalt are known but may be less common. The term "obsidian" is generally applied to large numbers of natural glasses that are rich in silica. Obsidian glasses may be classified into subcategories according to their silica content, with rhyolitic obsidians (containing typically about 73% $SiO_2$ by weight) being the most common.

Perlite is a hydrated natural glass that may contain, for example, about 72 to about 75% $SiO_2$, about 12 to about 14% $Al_2O_3$, about 0.5 to about 2% $Fe_2O_3$, about 3 to about 5% $Na_2O$, about 4 to about 5% $K_2O$, about 0.4 to about 1.5% CaO (by weight), and small amounts of other metallic elements. Perlite may be distinguished from other natural glasses by a higher content (such as about 2 to about 5% by weight) of chemically-bonded water, the presence of a vitreous, pearly luster, and characteristic concentric or arcuate onion skin-like (i.e., perlitic) fractures. Perlite may be expanded or non-expanded. Perlite products may be prepared by milling and thermal expansion, and may possess unique physical properties such as high porosity, low bulk density, and chemical inertness. Average particle size for the milled expanded perlite ranges from 5 to 200 microns, pore volume ranges from 2 to 10 L/mg with median pore size from 5 to 20 microns.

Pumice is a natural glass characterized by a mesoporous structure (e.g., having pores or vesicles with a size up to about 1 mm). The porous nature of pumice gives it a very low apparent density, in many cases allowing it to float on the surface of water. Most commercial pumice contains from about 60% to about 70% $SiO_2$ by weight. Pumice may be processed by milling and classification, and products may be used as lightweight aggregates and also as abrasives, adsorbents, and fillers. Unexpanded pumice and thermally-expanded pumice may also be used as filtration components.

According to the present invention, the silica or silicate mineral is diatomaceous earth. Diatomaceous earth products may be obtained from diatomaceous earth (also called "DE" or "diatomite" or "kieselgur"), which is generally known as a sediment-enriched in biogenic silica (i.e., silica produced or brought about by living organisms) in the form of siliceous skeletons (frustules) of diatoms. Diatoms are a diverse array of microscopic, single-celled, golden-brown algae or algae-like plants generally of the class *Bacillariophyceae* that possess an ornate siliceous skeleton of varied and intricate structures including two valves that, in the living diatom, fit together much like a pill box. Diatomaceous earth may form from the remains of water-borne diatoms and, therefore, diatomaceous earth deposits may be found close to either current or former bodies of water. Those deposits are generally divided into two categories based on source: freshwater and saltwater. Freshwater diatomaceous earth is generally mined from dry lakebeds and may be characterized as having a low crystalline silica content and a high iron content. In contrast, saltwater diatomaceous earth is generally extracted from oceanic areas and may be characterized as having a high crystalline silica content and a low iron content.

Diatomaceous earth is principally composed of the silica microfossils of aquatic unicellular algae known as diatoms. Diatomaceous earth typically has a chemical composition in the range of about 60 to 95% silica, 1 to 12% alumina and 0.5 to 8% iron oxide. It may also contain small amounts of other compounds such as calcium oxide, titanium dioxide, magnesium oxide, sodium oxide and potassium oxide. Diatomaceous earth has a highly porous structure, for example containing up to 80 to 90% voids, and consists of particles of a wide variety of shapes and sizes. In one embodiment, natural diatomaceous earth comprises about 90% $SiO_2$ mixed with other substances. In another embodiment, crude diatomaceous earth comprises about 90% $SiO_2$, plus various metal oxides, such as but not limited to Al, Fe, Ca, and Mg oxides.

The diatomaceous earth starting material may have any of various appropriate forms now known to the skilled artisan or hereafter discovered. In one embodiment, the at least one natural diatomaceous earth is unprocessed (e.g., not subjected to chemical and/or physical modification processes). Without wishing to be bound by theory, the impurities in natural diatomaceous earth, such as clays and organic matters, may, in some embodiments, provide higher cation exchange capacity. The diatomaceous earth may, for example, have an average particle size ranging from about 5 to about 200 microns. The diatomaceous earth may, for example, have a surface area ranging from 1 to 80 m$^2$/g. The diatomaceous earth may, for example have a pore volume ranging from 2 to 10 L/mg with a media pore size from 1 to 20 microns.

Diatomaceous earth may have a low cristobalite content. For example, the cristobalite content may be less than about 2% by weight. For example, the cristobalite content may be less than about 1% by weight. For example, the cristobalite content may be less than about 0.5% by weight. For example, the cristobalite content may be less than about 0.1% by weight. Cristobalite content may be measured by any appropriate measurement technique now known to the skilled artisan or hereafter discovered, including the specific method described in WO 2010/042614.

The diatomaceous earth may comprise at least one soluble metal. As used herein, the term "soluble metal" refers to any metal that may be dissolved in at least one liquid. Soluble metals are known to those of skill in the art and include, but are not limited to, iron, aluminium, calcium, vanadium, chromium, copper, zinc, nickel, cadmium, and mercury. When a filter aid comprising diatomaceous earth is used to filter at least one liquid, at least one soluble metal may dissociate from the diatomaceous earth filter aid and enter the liquid. Any appropriate protocol or test for measuring levels of at least one soluble metal in diatomaceous earth products may be used, including those now known to the skilled artisan or hereafter discovered. For example, the brewing industry has developed at least one protocol to measure the BSI of diatomaceous earth filter aids. BSI, or beer soluble iron, refers to the iron content, which may be measured in parts per million, of a filter aid comprising diatomaceous earth that dissociates in the presence of a liquid, such as beer. The European Beverage Convention (EBC) method contacts liquid potassium hydrogen phthalate with the filter aid and then analyzes the liquid for iron content. More specifically, the EBC method uses, for example, a 10 g/L solution of potassium hydrogen phthalate (KHP, $KHC_8H_4O_4$) as the extractant along with a given quantity of filter aid material, with a total contact time of two hours. Extracts are then analyzed for iron concentration by the FERROZINE method.

Diatomaceous earth, may have a permeability suitable for use in a filter aid composition. Permeability may be measured by any appropriate measurement technique now known to the skilled artisan or hereafter discovered. Permeability is generally measured in darcy units or darcy, as determined by the permeability of a porous bed 1 cm high and with a 1 cm$^2$ section through which flows a fluid with a viscosity of 1 mPa•s with a flow rate of 1 cm$^3$/sec under an applied pressure differential of 1 atmosphere. The principles for measuring permeability have been previously derived for porous media from Darcy's law (see, for example, J. Bear, "The Equation of Motion of a Homogeneous Fluid: Derivations of Darcy's Law," in Dynamics of Fluids in Porous Media 161-177 (2nd ed. 1988)). An array of devices and methods are in existence that may correlate with permeability. In one exemplary method useful for measuring permeability, a specially constructed device is designed to form a filter cake on a septum from a suspension of filtration media in water; the time required for a specified volume of water to flow through a measured thickness of filter cake of known cross-sectional area is measured. Thus, in an embodiment, the product described herein may have a permeability of at least 1.0 Da, preferably at least 3.0 Da.

Also described herein, synthetic silica-based minerals include, for example, fumed silica, silica fume, precipitated silica and silica gel. In certain examples, the silica-based mineral is not a synthetic silica.

Silicate-based minerals include both naturally-derived silicate-based minerals and synthetic silicate-based minerals. Naturally-derived silicate-based minerals include, for example, orthosilicates (e.g. andalusite, mullite), sorosilicates, cyclosilicates (e.g. bentonite, tourmaline), inosilicate (e.g. ferrolsilite, wollastonite), phyllosilicates (e.g. chrystolite, clays such as halloysite, kaolinite, montmorillonite, vermiculite, talc and pyrophyllite, mica) and tectosilicates (e.g. feldspar). The silicate-based mineral may, for example, be any one or more of these silicates. For example, the silicate-based mineral may be calcium silicate, magnesium silicate or combinations thereof.

Naturally-derived silicate-based minerals also include minerals that are made from a naturally occurring mineral. For example, calcium silicate may be made by reacting calcium oxide with a naturally-occurring silica- or silicate-based mineral. For example, calcium silicate may be made by reacting calcium oxide with diatomaceous earth. Calcium silicate that is made by reacting calcium oxide with diatomaceous earth may, for example, have a diatom-type mineral structure. The calcium silicate may, for example, have a pore volume equal to or greater than about 5.5 mL/g, for example equal to or greater than about 6.0 mL/g.

In one example, the mineral undergoes minimal processing following mining or extraction. In a further example, the mineral is subjected to at least one physical modification process. The skilled artisan will readily know physical modification processes appropriate for use, which may be now known or hereafter discovered; appropriate physical modification processes include, but are not limited to, milling, drying, and air classifying. In yet another embodiment, the mineral is subjected to at least one chemical modification process. The skilled artisan will readily know chemical modification processes appropriate for use in the present inventions, which may be now known or hereafter discovered; appropriate chemical modification processes include but are not limited to, silanization and calcination.

According to the present invention, the mineral is diatomaceous earth.

The composite materials disclosed herein have a particle size. Particle size may be measured by any appropriate measurement technique now known to the skilled artisan or hereafter discovered. In one exemplary method, particle size and particle size properties, such as particle size distribution ("psd"), are measured using a Leeds and Northrup Microtrac X100 laser particle size analyzer (Leeds and Northrup, North Wales, Pennsylvania, USA), which can determine particle size distribution over a particle size range from 0.12 $\mu$m to 704 $\mu$m. The size of a given particle is expressed in terms of the diameter of a sphere of equivalent diameter that sediments through the suspension, also known as an equivalent spherical diameter or "esd." The median particle size, or $d_{50}$ value, is the value at which 50% by weight of the particles have an esd less than that $d_{50}$ value. The $d_{10}$ value is the value at which 10% by weight of the particles have an esd less than that $d_{10}$ value. The $d_{90}$ value is the value at which 90% by weight of the particles have an esd less than that $d_{90}$ value.

The silica- or silicate-based mineral may, for example, have a $d_{10}$ ranging from about 0.5 to about 150 $\mu$m. For example, the mineral may have a $d_{10}$ ranging from about 0.5 to about 130 $\mu$m, for example from about 0.5 to about 120 $\mu$m, for example from about 0.5 to about 100 $\mu$m, for example from about 0.5 to about 50 $\mu$m, for example from about 0.5 to about 30 $\mu$m, for example from about 0.5 to about 20 $\mu$m, for example from about 0.5 to about 10 $\mu$m, for example from about 0.5 to about 5 $\mu$m. For example, the mineral may have a $d_{10}$ ranging from about 1 to about 10 $\mu$m, for example from about 2 to about 10 $\mu$m, for example from about 2 to about 20 $\mu$m. For example, the mineral may have a $d_{10}$ ranging from about 80 to about 150 $\mu$m, for example from about 90 to about 140 $\mu$m, for example from about 90 to about 130 $\mu$m, for example from about 100 to about 120 $\mu$m.

The silica- or silicate-based mineral may, for example, have a $d_{50}$ ranging from about 5 to about 450 $\mu$m. For example, the mineral may have a $d_{50}$ ranging from about 5 to about 100 $\mu$m, for example from about 5 to about 80, for example from about 5 to about 60 $\mu$m, for example from about 5 to about 50 $\mu$m, for example from about 5 to about 40 $\mu$m, for example from about 5 to about 30 $\mu$m, for example from about 5 to about 20 $\mu$m. For example, the mineral may have a $d_{50}$ ranging from about 20 to about 180 $\mu$m, for example from about 30 to about 170 $\mu$m, for example from about 40 to about 160 $\mu$m. For example, the mineral may have a $d_{50}$ ranging from about 200 to about 450 $\mu$m, for example from about 250 to about 450 $\mu$m, for example from about 300 to about 450 $\mu$m or from about 250 to about 400 $\mu$m.

The silica- or silicate based mineral may, for example, have a $d_{90}$ ranging from about 10 to about 1000 $\mu$m. For example, the mineral may have a $d_{90}$ ranging from about 15 to about 120 $\mu$m, for example from about 15 to about 100 $\mu$m, for example from about 15 to about 90 $\mu$m, for example from about 15 to about 80 $\mu$m, for example from about 15 to about 70 $\mu$m, for example from about 15 to about 60 $\mu$m, for example from about 15 to about 60 $\mu$m, for example from about 15 to about 50 $\mu$m, for example from about 15 to about 40 $\mu$m, for example from about 15 to about 30 $\mu$m, for example from about 20 to about 30 $\mu$m. For example, the mineral may have a $d_{90}$ ranging from about 40 to about 100 $\mu$m, for example from about 50 to about 90 $\mu$m, for example from about 50 to about 80 $\mu$m. For example, the mineral may have a $d_{90}$ ranging from about 100 to about 300 $\mu$m, for example from about 100 to about 200 $\mu$m, for example from about 100 to about 150 $\mu$m. For example, the mineral may have a $d_{90}$ ranging from about 600 to about 1000 $\mu$m, for example from about 600 to about 800 $\mu$m or from about 800 to about 1000 $\mu$m, for example from about 700 to about 900 $\mu$m.

In certain embodiments, the mineral is diatomaceous earth having a $d_{10}$ ranging from about 0.5 to about 150 $\mu$m. For example, the mineral may be diatomaceous earth having a $d_{10}$ ranging from about 0.5 to about 30 $\mu$m, for example from about 0.5 to about 20 $\mu$m, for example from about 0.5 to about 10 $\mu$m, for example from about 0.5 to about 5 $\mu$m. For example, the mineral may be spray-dried diatomaceous earth having a $d_{10}$ ranging from about 5 to about 150 $\mu$m, for example from about 5 to about 140 $\mu$m, for example from about 5 to about 130 $\mu$m, for example from about 5 to about 120 $\mu$m. For example, the mineral may be spray-dried diatomaceous earth having a $d_{10}$ ranging from about 5 to about 30 $\mu$m, for example from about 5 to about 25 $\mu$m, for example from about 5 to about 20 $\mu$m, for example from about 5 to about 15 $\mu$m. For example, the mineral may be granulated diatomaceous earth having a $d_{10}$ ranging from about 0.5 to about 10 $\mu$m, for example from about 0.5 to about 8 $\mu$m, for example from about 0.5 to about 6 $\mu$m, for example form about 0.5 to about 5 $\mu$m.

In certain embodiments, the mineral is diatomaceous earth having a $d_{50}$ ranging from about 5 to about 200 $\mu$m. For example, the mineral may be diatomaceous earth having a $d_{50}$ ranging from about 5 to about 60 $\mu$m, for example from about 5 to about 50 $\mu$m, for example from about 5 to about 40 $\mu$m, for example from about 5 to about 30 $\mu$m, for example from about 5 to about 20 $\mu$m, for example from about 5 to about 15 $\mu$m. For example, the mineral may be spray-dried diatomaceous earth having a $d_{50}$ ranging from about 20 to about 180 $\mu$m, for example from about 20 to about 170 $\mu$m, for example from about 20 to about 160 $\mu$m, for example from about 100 to about 180 $\mu$m. For example, the mineral may be spray-dried diatomaceous earth having a $d_{50}$ ranging from about 20 to about 60 $\mu$m, for example from about 20 to about 50 $\mu$m, for example from about 20 to about 40 $\mu$m, for example from about 30 to about 50 $\mu$m. For example, the mineral may be granulated diatomaceous earth having a $d_{50}$ ranging from about 10 to about 40 $\mu$m, for example from about 10 to about 30 $\mu$m, for example from about 15 to about 30 $\mu$m, for example form about 15 to about 25 $\mu$m.

In certain embodiments, the mineral is diatomaceous earth having a $d_{90}$ ranging from about 10 to about 300 $\mu$m. For example, the mineral may be diatomaceous earth having a $d_{50}$ ranging from about 15 to about 270 $\mu$m, for example from about 15 to about 260 $\mu$m, for example from about 15 to about 250 $\mu$m, for example from about 15 to about 100 $\mu$m, for example from about 15 to about 80 $\mu$m, for example from about 15 to about 60 $\mu$m, for example from about 15 to about 50 $\mu$m, for example from about 15 to about 40 $\mu$m, for example from about 15 to about 30 $\mu$m. For example, the mineral may be spray-dried diatomaceous earth having a $d_{50}$ ranging from about 50 to about 300 $\mu$m, for example from about 50 to about 280 $\mu$m, for example from about 50 to about 260 $\mu$m, for example from about 50 to about 250 $\mu$m. For example, the mineral may be spray-dried diatomaceous earth having a $d_{50}$ ranging from about 50 to about 100 $\mu$m, for example from about 50 to

about 90 μm, for example from about 50 to about 80 μm, for example from about 50 to about 70 μm. For example, the mineral may be granulated diatomaceous earth having a $d_{50}$ ranging from about 10 to about 50 μm, for example from about 20 to about 50 μm, for example from about 30 to about 50 μm, for example form about 30 to about 40 μm.

The mineral is able to absorb substances such as organic acid and/or oil and/or water. In certain embodiment, the mineral is able to absorb substances that are in suspension or solution with organic acid or oil or water.

The mineral has an organic acid absorption capacity equal to or greater than about 220 g/100 g of the mineral

In certain embodiments, the mineral has an oil-absorption capacity equal to or greater than about 220 g/100 g of the mineral.

The oil-absorption capacity and organic acid absorption capacity may vary between different minerals. For example, natural glasses such as perlite may have an oil-absorption capacity equal to or greater than about 270 g/100 g of the mineral. For example, natural glasses such as perlite may have an oil-absorption capacity equal to or greater than about 280 g/100 g of the mineral, for example equal to or greater than about 290 g/100 g of the mineral, for example equal to or greater than about 300 g/100 g of the mineral. For example, natural glasses such as perlite may have an oil-absorption capacity ranging from about 270 to about 800 g/100 g of the mineral, for example from about 270 to about 500 g/100 g of the mineral, for example from about 270 to about 400 g/100 g of the mineral.

For example, natural glasses such as perlite may have an organic acid absorption capacity equal to or greater than about 200 g/100 g of mineral. For example, natural glasses such as perlite may have an organic acid absorption capacity equal to or greater than about 300 g/100 g of mineral, for example equal to or greater than about 320 g/100 g of mineral, for example equal to or greater than about 340 g/100 g of mineral, for example equal to or greater than about 400 g/100 g of mineral. For example, natural glasses such as expanded, un-milled perlite may have an organic acid absorption capacity equal to or greater than about 500 g/100 g of mineral., for example equal to or greater than about 550 g/100 g of mineral, for example equal to or greater than about 600 g/100 g of mineral, for example equal to or greater than about 650 g/100 g of mineral, for example equal to or greater than about 700 g/100 g of mineral.

Diatomaceous earth has an oil-absorption capacity equal to or greater than about 220 g/100 g of the mineral. For example, diatomaceous earth may have an oil-absorption capacity equal to or greater than about 230 g/100 g of the mineral, for example equal to or greater than about 240 g/100 g of the mineral, for example equal to or greater than about 250 g/100 g of the mineral, for example equal to or greater than about 260 g/100 g of the mineral, for example equal to or greater than about 270 g/100 g of the mineral, for example equal to or greater than about 280 g/100 g of the mineral, for example equal to or greater than about 290 g/100 g of the mineral, for example equal to or greater than about 300 g/100 g of the mineral. For example, diatomaceous earth may have an oil-absorption capacity ranging from about 220 to about 600 g/100 g of the mineral, for example from about 220 to about 500 g/100 g of the mineral, for example from about 250 to about 400 g/100 g of the mineral, for example from about 300 to about 400 g/100 g of the mineral.

For example, diatomaceous earth may have an organic acid absorption capacity equal to or greater than about 250 g/100 g of mineral, for example equal to or greater than about 300 g/100 g of mineral, for example equal to or greater than about 350 g/100 g of mineral, for example equal to or greater than about 350 g/100 g of mineral.

According to the present invention, the mineral has a water-absorption capacity equal to or greater than about 200 g/100 g of the mineral. For example, the mineral may have a water-absorption capacity equal to or greater than 210 g/100 g of the mineral, for example equal to or greater than about 220 g/100 g of the mineral, for example equal to or greater than about 230 g/100 g of the mineral, for example equal to or greater than about 240 g/100 g of the mineral, for example equal to or greater than about 250 g/100 g of the mineral, for example equal to or greater than about 260 g/100 g of the mineral, for example equal to or greater than about 270 g/100 g of the mineral, for example equal to or greater than about 280 g/100 g of the mineral, for example equal to or greater than about 290 g/100 g of the mineral, for example equal to or greater than about 300 g/100 g of the mineral. For example, the mineral may have a water-absorption capacity equal to or greater than about 310 g/100 g of the mineral, for example equal to or greater than about 320 g/100 g of the mineral, for example equal to or greater than about 330 g/100 g of the mineral, for example equal to or greater than about 340 g/100 g of the mineral, for example equal to or greater than about 350 g/100 g of the mineral. For example, the mineral may have a water-absorption capacity equal to or greater than about 400 g/100 g of the mineral, for example equal to or greater than about 450 g/100 g of the mineral, for example equal to or greater than about 500 g/100 g of the mineral.

Diatomaceous earth has a water-absorption capacity equal to or greater than about 200 g/100 g of mineral. For example, diatomaceous earth may have a water-absorption capacity equal to or greater than about 250 g/100 g of mineral, for example equal to or greater than about 300 g/100 g of mineral, for example equal to or greater than about 350 g/100 g of mineral, for example equal to or greater than about 350 g/100 g of mineral.

The organic acid absorption capacity, oil-absorption capacity and water-absorption capacities are determined by weighing a sample of mineral into a container (e.g. 1 to 10 grams into a 100 to 300 mL ceramic or glass dish) and adding either organic acid, oil or water to the mineral gradually in a dropwise manner (e.g. about 1 drop per second). The sample is stirred during the addition of the liquid so that each drop falls on a dry portion of the mineral sample. When the sample particles become wet with water, they coalesce and form small lumps of paste. These lumps should be kept distributed throughout the mass, using a minimum of stirring, and using care not to use pressure in the mixing. As the absorption of water progresses, the lumps of paste form larger lumps which, when stirred around, form balls. When this point is reached, the rate and quantity of the water added should be decreased to ensure you do not go past the end point. When adding water at this point it should strike the balls, not the dry sample. These balls are stirred around to bring the watery surface into contact with the remaining dry sample. When the dry sample is wet and picked up, the paste lumps tend to smear on the sides and bottom of the casserole. This is the end point. The total amount of water used in noted and the g of liquid (organic acid/oil/water) per 100 g of mineral sample is calculated. The organic acid may, for example, be a blend of organic acids, for example a mixture of acetic acid and formic acid and propionic acid, for example the blend known as Fylax® available from Selko. The oil may, for example, be linseed oil. The oil absorption of the samples may be determined on a weight basis according to ASTM-D1483-95. The oil absorption in weight percentage may be calculated as follows:

$$\text{Oil Absorption (wt. \%)} = \frac{\text{Volume Oil Used (mL)} \times \text{Specific Gravity of Oil}}{\text{Weight of Sample (g)}} \times 100$$

In certain embodiments, the mineral is the form of a free-flowing granulate. "Free-flowing" means that the mineral can move freely, for example the mineral can move in a continuous stream (e.g. "poured"). "Granulate" means that the mineral is in the form of particles or grains (particles formed from more than one smaller particles).

Whether a mineral is in the form of a "free-flowing granulate" may be determined by its base flow energy (BFE). For example, a mineral may be considered to be a "free-flowing granulate" if it has a BFE equal to or less than about 1200 mJ. For example, a mineral may be considered to be "free-flowing" if it has a BFE equal to or less than about 1100 mJ, for example equal to or less than about 1000 mJ, for example equal to or less than about 800 mJ, for example equal to or less than about 700 mJ, for example equal to or less than about 600 mJ.

The mineral may be in the form of a free-flowing granulate even at relatively high oil and/or water contents.

In certain embodiments, the mineral has a base flow energy (BFE) equal to or less that is equal to or less than about 1200 mJ when the mineral has an organic acid content equal to or greater than about 220 g/100 g of the mineral.

In certain embodiments, the mineral has a base flow energy (BFE) that is equal to or less than about 1200 mJ when the mineral has a water content of 200 g/100 g of the mineral.

Base flow energy is measured using a Freeman Powder Rheometer model FT3. The FT3 rheometer drives a blade along a helical path downward through a powder sample. As the blade forces it way down through the powder the force imposed upon it is measured. It is this data that forms the basis of the measurements made. The helical path that the blade takes through the sample is determined by a combination of the rotational and axial speeds. Each particle within the powder mass lies at a state of rest until forced to move, coming to rest again as the blade moves on. The pattern of powder displacement is virtually steady state, allowing flow to be observed and generally resulting in smooth, linear or logarithmic profiles of the measured forces. These forces are those required to initiate shearing and breakdown of interparticulate bonding of the powder in the zone immediately around the blade, a process that is continuous.

The base flow energy is the energy required to displace a constant volume of conditioned powder at a given flow pattern and flow rate. Samples are prepared by measuring 160ml of powder into the sample vessel and recording the mass. A conditioning cycle is then carried out on the sample and the volume rechecked and adjusted to 160ml if necessary before conducting the test. The BFE test consists of a standard conditioning cycle with blade tip speed of 100mm/s in a 5°C negative upward helical path followed by a test cycle with a downward transverse tip speed set at 100mm/s and a 10° negative helix.

The specific pore volume of a packed body of the granular material may, for example, be at least about 3 cc/g, or at least about 4 cc/g or at least about 5 cc/g. Typically, products of the invention have little, if any, pore volume in pores smaller than 0.1 $\mu$m or larger than 100 $\mu$m. The majority of the pore volume, for example at least 70% of the pore volume may be in pores larger than 1 $\mu$m and smaller than 100 $\mu$m. At least 40% of the pore volume may be in pores larger than 10 $\mu$m and smaller than 100 $\mu$m. Pore volume may be measured by mercury porosimetry using a CE Instruments Model "Pascal 240" mercury porosimeter. The method involves evacuation of the sample placed in a dilatometer, which is subsequently filled with mercury. Pressure is applied to the filled dilatometer and the mercury intrudes first into the intra-particle pores between granules and the hollow voids of the particles, and then into the pores of the granules within the sample under test. The volume of mercury intruded is determined by a precision capacitive electrode and the pore diameter calculated from the applied pressure according to the Washburn equation. The contact angle for porosimetery was 140°, and the pressure typically 0.012 MPa to 200 MPa. The average pore diameter of a packed body of the granular material may be of the order of 5-15 $\mu$m, for example about 10 $\mu$m. Typically, the average pore diameter of the granules (excluding intra-particle pores and the hollow void formed with the granules) is of the order of 1-3 $\mu$m, for example about 2 $\mu$m. The mineral may, for example, particularly have a pore volume as described where the mineral is diatomaceous earth.

The mineral may have a surface area equal to or greater than about 1 $m^2$/g. For example, the mineral may have a surface area equal to or greater than about 2 $m^2$/g or equal to or greater than about 5 $m^2$/g or equal to or greater than about

10 m$^2$/g or equal to or greater than about 20 m$^2$/g or equal to or greater than about 30 m$^2$/g or equal to or greater than about 40 m$^2$/g or equal to or greater than about 50 m$^2$/g or equal to or greater than about 60 m$^2$/g or equal to or greater than about 70 m$^2$/g. For example, the mineral may have a surface area ranging from about 1 m$^2$/g to about 100 m$^2$/g or from about 2 m$^2$/g to about 90 m$^2$/g or from about 5 m$^2$/g to about 80 m$^2$/g.

The surface area of the samples may be determined according to the BET method by the quantity of nitrogen adsorbed on the surface of said particles so to as to form a monomolecular layer completely covering said surface (measurement according to the BET method, AFNOR standard X11-621 and 622 or ISO 9277). In certain embodiments, specific surface is determined in accordance with ISO 9277, or any method equivalent thereto.

In certain embodiments, the mineral has a L whiteness value equal to or greater than about 80. For example, the mineral may have a L whiteness value equal to or greater than about 82 or equal to or greater than about 84 or equal to or greater than about 85 or equal to or greater than about 86 or equal to or greater than about 88 or equal to or greater than about 90 or equal to or greater than about 92 or equal to or greater than about 94. For example, the mineral may have a L whiteness value ranging from about 80 to about 100 or from about 82 to about 98 or from about 84 to about 96 or from about 85 to about 95.

L, a and b may be determined using the Hunter scale collected on a Spectro/plus Spectrophotometer (Colour and Appearance Technology, Inc., Princeton, New Jersey) as described in the Examples below.

Also described herein, the mineral may be spray-dried (i.e. the product of a spray-drying process). The spray-dried mineral product may, for example, be treated by one or more physical or chemical modification processes, such as milling, drying, air classifying, silanization and calcinations.

The spray-dried mineral may, for example, comprise substantially spherical granules. For example, greater than about 50 wt% of the spray-dried mineral, for example greater than about 60 wt%, for example greater than about 70 wt%, for example greater than about 80 wt%, for example greater than about 90 wt% of the spray-dried mineral may comprise substantially spherical granules. For example, each substantially spherical granule may have a mineral shell surrounded by a hollow core. The product may, for example, have substantially the same form after any of the physical or chemical modification processes described above, for example after calcinations.

The spray-dried mineral may, for example, further comprise a binder, which may, for example, have been included in the suspension that was spray-dried to facilitate the formation of spray-dried granules. A binder that remains associated with or in the product may be referred to as a "permanent binder". Examples of permanent binders are cross-linked alginates, thermosetting resins, thermoplastic resins and styrene-butadiene polymers. The binder may, for example, be present in the spray-dried mineral in an amount equal to or less than about 10 wt%, for example equal to or less than about 8 wt%, for example equal to or less than about 6 wt%, for example equal to or less than about 5 wt%, for example equal to or less than about 4 wt%, for example equal to or less than about 3 wt%, for example equal to or less than about 2 wt%, for example equal to or less than about 1 wt%.

The spray-drying process may yield uniform, or substantially uniform, spray-dried granules, in which case the diameter of the granules will lie in the aforesaid range. The steepness of the particle size distribution curve, as characterized by the $d_{90}/d_{10}$ ratio, is typically at least 5, preferably at least 8. In some examples, the spray-dried granulate may be essentially mono-disperse.

In certain examples, the spray-dried mineral may have a $d_{10}$ ranging from about 1 to about 150 $\mu$m, for example from about 2 to about 140 $\mu$m, for example from about 2 to about 130 $\mu$m, for example from about 2 to about 120 $\mu$m. For example, the spray-dried mineral may have a $d_{10}$ ranging from about 1 to about 30 $\mu$m, for example from about 1 to about 25 $\mu$m, for example from about 1 to about 20 $\mu$m. For example, the spray-dried mineral may have a $d_{10}$ ranging from about 80 to about 150 $\mu$m, for example from about 80 to about 140 $\mu$m, for example from about 80 to about 130 $\mu$m, for example from about 80 to about 120 $\mu$m, for example from about 90 to about 110 $\mu$m.

In certain examples, the spray-dried mineral may have a $d_{50}$ ranging from about 10 to about 200 $\mu$m, for example from about 15 to about 180 $\mu$m, for example from about 20 to about 160 $\mu$m. For example, the spray-dried mineral may have a $d_{50}$ ranging from about 10 to about 60 $\mu$m, for example from about 15 to about 55 $\mu$m, for example from about 15 to about 50 $\mu$m, for example from about 20 to about 40 $\mu$m, for example from about 20 to about 30 $\mu$m. For example, the spray-dried mineral may have a $d_{50}$ ranging from about 100 to about 200 $\mu$m, for example from about 120 to about 180 $\mu$m, for example from about 130 to about 180 $\mu$m, for example from about 140 to about 170 $\mu$m, for example from about 140 to about 160 $\mu$m, for example from about 150 to about 160 $\mu$m.

In certain examples, the spray-dried mineral may have a $d_{90}$ ranging from about 20 to about 300 $\mu$m, for example from about 30 to about 290 $\mu$m, for example from about 40 to about 280 $\mu$m, for example from about 50 to about 270 $\mu$m, for example from about 50 to about 260 $\mu$m. For example, the spray-dried mineral may have a $d_{90}$ ranging from about 20 to about 100 $\mu$m, for example from about 30 to about 90 $\mu$m, for example from about 40 to about 80 $\mu$m, for example from about 50 to about 80 $\mu$m. For example, the spray-dried mineral may have a $d_{90}$ ranging from about 200 to about 300 $\mu$m, for example from about 210 to about 290 $\mu$m, for example from about 220 to about 280 $\mu$m, for example from about 230 to about 270 $\mu$m, for example from about 240 to about 260 $\mu$m.

In certain examples, the spray-dried mineral may have an organic acid-absorption capacity ranging from about 300 to about 400 g/100 g of the mineral, for example from about 320 to about 380 g/100 g of the mineral, for example from about 340 to about 380 g/100 g of the mineral. In certain embodiments, the spray-dried mineral may have a water-absorption capacity ranging from about 250 to about 400 g/100 g of the mineral, for example from about 250 to about 380 g/100 g of the mineral, for example from about 250 to about 360 g/100 g of the mineral.

In certain examples, the mineral is agglomerated (granulated) with a binder, wherein one or more smaller particles are attached to form a larger particle. The binder may, for example, be any of the binders described herein, which may also be used for spray drying. For example, the binder may be polyvinyl alcohol. For example, the binder may be partly saponified. For example, the binder may have a viscosity of about 5 cps. For example, the binder may have a % hydrolysation equal to or greater than about 80 %, for example equal to or greater than about 85 %, for example about 88 %. For example, the binder may be Celvol 205E®, available from Sekisui. For example, the binder may be used in an amount equal to or less than about 10 % based on the total weight of the mineral. For example, the binder may be used in an amount ranging from about 0.5 to about 8 %, for example from about 1 to about 6 %, for example from about 1 to about 5 %, for example from about 1 to about 3 %, based on the total weight of the mineral. For example, the binder may be used in an amount equal to or less than about 5 wt%, for example equal to or less than about 4 wt%, for example equal to or less than about 3 wt%.

The mineral may, for example, be agglomerated by any one or more of the methods described below in relation to making the composites comprising first and second silica- or silicate-based minerals. The mineral may, for example, be agglomerated by mixing the mineral with a binder, for example using an Eirich mixer or a food mixer (e.g. Hobart food mixer) or using a pelletizer such as a pan pelletizer or drum pelletizer etc. The mineral may, for example, be agglomerated by spray-drying. The mineral may, for example, be agglomerated by spray-drying the binder onto the mineral. For example, a 100 g solution of 3 wt% polyvinyl alcohol may be sprayed onto 100 g of the mineral (e.g. diatomaceous earth). The mineral may, for example, be agglomerated by precipitating the binder onto the mineral in situ.

Methods of Spray-Drying

There is also provided a method of making any of the minerals described herein. For example, the method may involve spray-drying any of the minerals described herein. In certain embodiments, spray-drying a mineral may improve the oil and/or water-absorption properties of the mineral. Thus, there is also provided herein a method of improving the oil- and/or water-absorption properties of the mineral (e.g. a method of increasing the oil- and/or water-absorption properties (e.g. the oil- and/or water-absorption capacities) of the mineral).

Spray-drying a mineral may independently increase each of the organic acid- and/or oil- and/or water-absorption capacity of the mineral by at least about 10 g/100 g of the mineral. For example, spray-drying may independently increase each of the organic acid- and/or oil- and/or water-absorption capacity of the mineral by at least about 20 g/100 g of the mineral, for example by at least about 25 g/100 g of the mineral, for example at least about 30 g/100 g of the mineral, for example at least about 35 g/100 g of the mineral, for example at least about 40 g/100 g of the mineral, for example at least about 45 g/100 g of the mineral, for example at least about 50 g/100 g of the mineral, for example at least about 55 g/100 g of the mineral, for example at least about 60 g/100 g of the mineral. For example, spray-drying may independently increase each of the organic acid- and/or oil- and/or water-absorption capacity from about 10 to about 150 g/100 g of the mineral, for example from about 15 to about 150 g/100 g of the mineral, for example from about 20 to about 150 g/100 g of the mineral, for example from about 20 to about 120 g/100 g of the mineral, for example from about 20 to about 100 g/100 g of the mineral, for example from about 25 to about 90 g/100 g of the mineral.

Spray-drying is a method of producing a dry powder from a liquid or slurry by rapidly drying using a hot gas. The methods described herein comprise a step in which a suspension comprising particles of mineral is spray-dried. A mineral granulate is recovered. The recovered granulate may be heat treated (also referred to herein as "calcined").

For example, the methods described herein may comprising spray-drying a suspension comprising particles of a mineral (e.g. a mineral as described herein), a liquid medium and a binder and recovering a spray-dried mineral granulate. The mineral starting material and/or the spray-dried mineral granulate may have any one or more of the characteristics described herein. For example, the spray-dried mineral granulate may have an increased organic acid- and/or oil- and/or water-absorption capacity in comparison to the organic acid- and/or oil- and/or water-absorption capacity of the mineral prior to the spray-drying step.

The suspension which is to be spray-dried is typically an aqueous suspension comprising a liquid medium and a solids portion. The liquid medium is typically water.

The suspension may further include a binder. The binder may be inorganic or organic and may comprise a solid component, as for example a latex type binder. The binder may be included in the suspension to facilitate the formation of spray-dried granules.

In an example, the binder may be a temporary binder. By "temporary binder" is meant a binder which is not intended to remain in the product but acts to bind particles of the mineral together and support the spray-dried body after initial formation, which can then be subjected to one or more further treatment steps, including steps intended to impart structural rigidity to the spray-dried bodies, such as a heat treatment. Such temporary binders may thus be thermally fugitive, that is to say are removed from the spray-dried bodies on the application of sufficient heat which may vaporize or burn the binder material. Examples of suitable temporary binders are starches, carbohydrates, sugars, poly-vinyl acetates, poly-vinyl alcohols, latex, gelatines, waxes, celluloses, dextrines, thermo-plastic resins, thermo-setting resins, chlorinated hydrocarbons, gums, flours, caseins, alginates, proteins, bitumens, acrylics, epoxy resins, and urea. In embodiments of the invention, the temporary binder may be a poly vinyl alcohol binder or a latex binder. The amount of temporary binder in the suspension may be in the range of up to 10 wt% on a solids basis, for example 2-10 wt%. Where the binder is a temporary binder, the spray-dried granulate may be subjected to a heat treatment, or calcination, step in order to impart structural rigidity to the spray-dried bodies. In the heat treatment step, the temporary binder is removed, or substantially removed, from the spray-dried bodies.

In another example, the binder may be a permanent binder. By "permanent binder" is meant a binder which is intended to remain in the product and provide structural strength to the spray-dried bodies without the need for a high temperature calcination step. Examples of permanent binders are cross-linked alginates, thermosetting resins, thermo-plastic resins and styrene-butadiene polymers. The specific permanent binder to be used may be selected to ensure that the binder provides structural support to the aggregate without being significantly soluble in the liquid to be filtered. For example a binder which is insoluble in water would be suitable for use in a filter medium which is to be used in beer filtration.

The permanent binder may also, for example, be cross-linkable. In case such cross-linkable binders are used, a further chemical or low temperature heat treatment (for example less than 200°C) may be required after the spray-dried bodies are formed in order to effect cross-linking. An example of a suitable cross-linkable binder is a copolymer of a vinyl acetate and an acrylic ester, such as Vinnapas AN214 from Wacker Chemie. It is to be appreciated that permanent binders used in the present invention may be thermally fugitive, if organic in nature. However, a distinction between a temporary binder and a permanent binder which is thermally fugitive is that a permanent binder is capable of fixing the aggregated structure produced during the spray-drying step, without the need for a calcination treatment.

Other permanent binders which are not thermally fugitive may be used. Such binders are inorganic-based. Examples include cements, pozzolanic materials, silcates, waterglass, gypsums, bentonites, and borates. Also included are aluminate binders, including alkali metal aluminate binders such as sodium aluminate, potassium aluminate or lithium aluminate, and alkaline earth metal aluminate binders, such as calcium aluminate and magnesium aluminate. An advantage of using a permanent binder is that a calcination step can be avoided.

The solids portion of the suspension comprises the particulate mineral component together with one or more optional additional inorganic components and one or more optional organic solid components.

The inorganic solids content of the suspension is dependent on the spray-drying method to be used, which is discussed in more detail below, and the size of spray-dried granules desired. Typically, however, in order to have a viscosity suitable for spray-drying, the suspension should have an inorganic solids content of at least 5%, for example at least 10%, for example at least 15% by weight, based on the weight of the suspension, and may have an inorganic solids content of up to 30%, or 25% or 20%, based on the weight of the suspension. Typically, the solids content will be in the range of 15-25% by weight, based on the weight of the suspension.

The optional inorganic component may comprise one or more particulate inorganic mineral in addition to the mineral as described herein; and/or one or more suitable fluxing agent. The optional organic solids component may be the solids component of an organic binder.

A small amount of an additional inorganic mineral component, for example 20% or less, based on the total weight of the inorganic solids in the suspension, for example 10% or less or 5% or less, based on the total weight of the inorganic solids present in the suspension may be included in the suspension that is spray-dried. This will result in spray-dried granules including the additional inorganic mineral component, for example in the outer wall thereof. This may be used to adjust the properties of the spray-dried granules, for example strength and/or permeability. Examples of additional inorganic mineral components are natural or synthetic silicate or aluminosilicate materials, unimproved diatomaceous earth, saltwater diatomaceous earth, expanded perlite, pumicite, natural glass, cellulose, activated charcoal, feldspars, nepheline syenite, sepiolite, zeolite, and clay. Examples of clay minerals are halloysite, kaolinite and bentonite.

A fluxing agent is an optional additional component of the suspension that is spray-dried. A fluxing agent may be necessary where the spray-dried granules are to be calcined (so-called "flux-calcining"). The presence of at least one fluxing agent during calcination may reduce the temperature at which mineral particles in the wall of the spray-dried bodies are caused to be sintered together.

Suitable agents as the fluxing agent are any now known to those skilled in the art or which may hereafter be discovered. In one embodiment, the fluxing agent is sodium carbonate (soda ash, $Na_2CO_3$). In another embodiment, the fluxing agent is sodium hydroxide (NaOH). In a further embodiment, the at least one fluxing agent is sodium chloride (NaCl). In yet another embodiment, the at least one fluxing agent is potassium carbonate ($K_2CO_3$). In yet a further embodiment, the at least one fluxing agent is sodium borate ($Na_2B_4O_7$).

In one embodiment, the fluxing agent is at least one salt of at least one alkali metal in Group IA. In another embodiment, the fluxing agent is at least one salt of at least one alkali metal. In a further embodiment, the at least one alkali metal is sodium. In yet another embodiment, the at least one alkali metal is chosen from alkali metals having a larger atomic radius than that of sodium. In yet a further embodiment, the at least one alkali metal is potassium. In still another embodiment, the at least one alkali metal is rubidium.

The at least one fluxing agent is added to the suspension before spray-drying; as a result, the fluxing agent is located within the wall of the spray-dried granules at locations where it is readily able to provide its fluxing function.

The fluxing agent may be present in the suspension in an amount of less than about 8% based on the total weight of inorganic solids in the suspension, or in an amount of less than about 7%, or an amount of less than about 6%, or in amount of less than about 5%, or in amount of less than about 4%, or in amount of less than about 3%, or in amount less than about 2%. In another embodiment, the suspension contains from about 0.5% to about 10% fluxing agent, based on the total weight of inorganic solids in the suspension.

In some examples where the spray-dried granules are flux-calcined, the at least one fluxing agent may undergo a chemical decomposition reaction. In one embodiment of such a chemical decomposition, at least one fluxing agent containing sodium bonds with diatom silica present in the at least one feed material to form sodium silicate, expelling carbon dioxide gas in the process. In another embodiment, at least one fluxing agent containing at least one alkaline metal bonds with diatom silica present in the at least one feed material to form at least one alkaline metal silicate.

The suspension may be spray-dried in a manner which is known per se. The suspension may be fed to the inlet of a spray-dryer and spray-dried material is discharged from the atomiser.

Spray-drying may also be carried out using a nozzle atomiser or fountain spray-drying technique, in which the slurry is sprayed upwards from the cone of the drying chamber. This allows drying to take place during the complete flight-arc of the droplets before they return to the bottom of the dryer, providing a coarser, more free-flowing powder.

Another type of spray-dryer which may be used in the invention is one which employs a "rotating wheel" or "spinning disc" atomiser.

One example of a suitable spray-drying apparatus is a Niro Minor spray dryer unit. This machine has a drying chamber 800mm in diameter, 600mm cylindrical height being conical based and is fitted with an air driven disc type atomiser. The atomiser may be run at a speed of 30,000rpm. Drying may be carried out using an inlet-air temperature of 300°C. Slurry is fed via a peristaltic pump to the atomiser at a rate selected to maintain the required outlet temperature (typically 110 to 120°C).

In one example of a method of spray-drying, an inlet temperature between 350 and 400 °C and an outlet temperature between 110 and 120°C was used.

The inorganic solids content of the suspension is dependent on the spray-drying method to be used, which is discussed in more detail below, and the size of spray-dried granules desired. Typically, however, the suspension will have an inorganic solids content of the order of 5 to 30 wt%, for example 15 to 25 wt%.

The heat treatment, also referred to herein as a calcination treatment may be carried out at a suitable temperature to cause mineral particles in the wall of the spray-dried bodies to be sintered together and thus result in a body which is resistant to crushing. The maximum calcination temperature may be for example at least 500°C, or at least 600°C, or at least 700°C, or at least 800°C, or at least 900°C. In order to avoid destroying the fine structure of the spray-dried bodies and incurring additional cost, the maximum calcination temperature is typically less than 1200°C, for example less than 1100°C or less than 1000°C.

The duration of calcination can be determined empirically depending on the desired outcome. However, typically calcination may be carried out such that the duration at peak temperature is less than four hours, or less than three hours, or less than two hours, or less than one hour. In an embodiment, calcination may be carried out by "flash" calcination, in which the calcination is conducted very rapidly. Calcination may be carried out in a batch process, or in a continuous process. A suitable continuous process may use a rotary tube furnace, in which the uncalcined feed material is continuously passed through a heated zone maintained at the appropriate temperature. In embodiments, the calcination may be carried out by increasing the calcination temperature at a rate of, for example, between 1 and 50°C per minute, for example 1 to 10°C per minute, up to the final, maximum temperature and then cooled at a rate of, for example, 1 to 50°C per minute, for example 5 to 20°C per minute, to room temperature.

The calcined, spray-dried granulate has substantially the same particle size distribution as the uncalcined starting material.

According to the present invention, the silica or silicate mineral is diatomaceous earth.

Methods of Making the Mineral Composites

There is provided herein a method of making the mineral composites disclosed herein. Also described herein, the method comprises combining a first silica- or silicate-based mineral with a second silica- or silicate-based mineral that is different from the first mineral and any other optional components of the composite.

The first and second mineral components may be combined by mixing and/or blending by any technique known to those skilled in the art.

The first and second mineral components may be co-agglomerated. The co-agglomeration may, for example, comprise adding a binder to the first and/or second silica- or silicate-based minerals or adding a binder to a blend of the first and second silica- or silicate-based minerals.

The binder may, for example, be any of the binders described herein, which may also be used for spray drying. For example, the binder may be polyvinyl alcohol. For example, the binder may be partly saponified. For example, the binder may have a viscosity of about 5 cps. For example, the binder may have a % hydrolysation equal to or greater than about 80 %, for example equal to or greater than about 85 %, for example about 88 %. For example, the binder may be Celvol 205E®, available from Sekisui. For example, the binder may be used in an amount equal to or less than about 10 % based on the total weight of the mineral. For example, the binder may be used in an amount ranging from about 0.5 to about 8 %, for example from about 1 to about 6 %, for example from about 1 to about 5 %, for example from about 1 to about 3 %, based on the total weight of the mineral. For example, the binder may be used in an amount equal to or less than about 5 wt%, for example equal to or less than about 4 wt%, for example equal to or less than about 3 wt%.

In certain examples, the binder may, for example, be a precipitated binder. The binder may, for example, be a silica binder. For example, the binder may be an alkali silica binder. For example, the binder may be sodium silicate and/or potassium silicate. Without wishing to be bound by theory, co-agglomeration of the first and second silica- or silicate-based minerals may result in the first and second mineral components attaching to one another to form larger particles relative to a blend of the first and second mineral components.

In certain examples, the method comprises preparing an aqueous solution of the binder (e.g. silica binder) and contacting the aqueous solution with the first and/or second silica- or silicate-based mineral or a blend of the first and second silica- or silicate-based minerals. One or more agglomerations may be performed, for example, when multiple binders (e.g. multiple silica binders) and/or multiple minerals or mineral blends are used.

In some examples, contacting includes mixing the binder solution with a blend of the minerals. In some embodiments, the mixing includes agitation. In some embodiments, the mineral blend is mixed sufficiently to at least substantially uniformly distribute the binder solution among the agglomeration points of contact of the mineral components. In some examples, the mineral blend and the binder solution are mixed with sufficient agitation to at least substantially uniformly distribute the binder solution among the agglomeration points of contact of the blend of the mineral components without damaging the structure of the mineral components. In some embodiments, the contacting includes low-shear mixing. The mineral may, for example, be agglomerated by mixing the mineral with a binder, for example using an Eirich mixer or a food mixer (e.g. Hobart food mixer) or using a pelletizer such as a pan pelletizer or drum pelletizer etc.

In some examples, mixing occurs for about one hour. In other embodiments, mixing occurs for less than about one hour. In further examples, mixing occurs for about 30 minutes. In yet other examples, mixing occurs for about 20 minutes. In still further examples, mixing occurs for about 10 minutes.

In some examples, mixing occurs at about room temperature (i.e., from about 20°C to about 23°C). In other examples, mixing occurs at a temperature of from about 20°C to about 50°C. In further examples, mixing occurs at a temperature of from about 30°C to about 45°C. In still other examples, mixing occurs at a temperature of from about 35°C to about 40°C.

According to some examples, contacting includes spraying the mineral blend of with at least one binder solution. In some examples, the spraying is intermittent. In other examples, the spraying is continuous. In further examples, spraying includes mixing the blend while spraying with the at least one binder solution, for example, to expose different agglomeration points of contacts to the spray. In some examples, such mixing is intermittent. In other embodiments, such mixing is continuous. In some examples, the a mixture of mineral blend and binder is spray-dried together.

In some examples, the at least one binder is present in the binder solution in an amount from less than about 40% by weight, relative to the weight of the at least one binder solution. In some examples, the at least one binder ranges from about 1% to about 10% by weight. In further examples, the at least one binder ranges from about 1% to about 5% by weight.

The at least one aqueous solution of the at least one binder may be prepared with water. In some examples, the water is deionized water. In some examples, the water is ultrapure water. In further examples, the water has been treated to remove or decrease the levels of metals, toxins, and/or other undesirable elements before it is contacted with the at least one binder.

The amount of at least one aqueous solution contacted with the mineral blend may range from about 0.25 parts to about 1.5 parts of aqueous solution to one part blend. In some examples, about 1 part aqueous solution is contacted with about 1 part blend.

Before and/or after the agglomeration, the mineral components may be subjected to at least one classification step. For example, before and/or after at least one heat treatment, the mineral components may, in some examples, be subjected to at least one classification step. In some embodiments, the particle size of the mineral components is adjusted to a suitable or desired size using any one of several techniques well known in the art. In some examples, the mineral components are subjected to at least one mechanical separation to adjust the powder size distribution. Appropriate mechanical separation techniques are well known to the skilled artisan and include, but are not limited to, milling, grinding, screening, extrusion, triboelectric separation, liquid classification, aging, and air classification.

The mineral components and/or co-agglomerated mineral composite may be subjected to at least one heat treatment. Appropriate heat treatment processes are well-known to the skilled artisan, and include those now known or that may hereinafter be discovered. In some examples, the at least one heat treatment decreases the amount of organics and/or volatiles in the heat-treated mineral composition. In some examples, the at least one heat treatment includes at least one calcination. In some examples, the at least one heat treatment includes at least one flux calcination. In some examples, the at least one heat treatment includes at least one roasting.

Calcination may be conducted according to any appropriate process now known to the skilled artisan or hereafter discovered. In some examples, calcination is conducted at temperatures below the melting point of the mineral(s). In some embodiments, calcination is conducted at a temperature ranging from about 600°C to about 1100°C. In some examples, the calcination temperature ranges from about 600°C to about 700°C. In some examples, the calcination temperature ranges from about 700°C to about 800°C. In some examples, the calcination temperature ranges from about 800°C to about 900°C. In some examples, the calcination temperature is chosen from the group consisting of about 600°C, about 700°C, about 800°C, about 900°C, about 1000°C, and about 1100°C. Heat treatment at a lower temperature may result in an energy savings over other processes for the preparation of mineral composites.

Flux calcination includes conducting at least one calcination in the presence of at least one fluxing agent. Flux calcination may be conducted according to any appropriate process now known to the skilled artisan or hereafter discovered. In some examples, the at least one fluxing agent is any material now known to the skilled artisan or hereafter discovered that may act as a fluxing agent. In some examples, the at least one fluxing agent is a salt including at least one alkali metal. In some embodiments, the at least one fluxing agent is chosen from the group consisting of carbonate, silicate, chloride, and hydroxide salts. In other examples, the at least one fluxing agent is chosen from the group consisting of sodium, potassium, rubidium, and cesium salts. In still further examples, the at least one fluxing agent is chosen from the group consisting of sodium, potassium, rubidium, and cesium carbonate salts.

Roasting may be conducted according to any appropriate process now known to the skilled artisan or hereafter discovered. In some examples, roasting is a calcination process conducted at a generally lower temperature that helps to avoid formation of crystalline mineral (e.g. crystalline silica) in the mineral (e.g. diatomaceous earth and/or natural glass). In some examples, roasting is conducted at a temperature ranging from about 450°C to about 900°C. In some examples, the roasting temperature ranges from about 500°C to about 800°C. In some examples, the roasting temperature ranges from about 600°C to about 700°C. In some examples, the roasting temperature ranges from about 700°C to about 900°C. In some examples, the roasting temperature is chosen from the group consisting of about 450°C, about 500°C, about 600°C, about 700°C, about 800°C, and about 900°C.

According to some examples, the mineral components may be subjected to at least one heat treatment, followed by co-agglomerating the heat treated mineral components with at least one binder (e.g. silica binder).

Uses of the Minerals and Mineral Composites

There is further provided herein the various uses of the minerals and mineral composites disclosed herein.

For example, the minerals and mineral composites disclosed herein may be used to absorb organic acid and/or oil and/or water. For example, the minerals and mineral composites disclosed herein may be used to absorb substances that are in solution or suspension with organic acid and/or oil and/or water.

For example, the minerals and mineral composites disclosed herein may be used to absorb organic acid and/or oil and/or water or substances that are in solution or suspension with oil and/or water in a form that is free-flowing. This may be advantageous, for example, for administration of the organic acid and/or oil and/or water substance.

For example, the minerals and mineral composites disclosed herein may be used as a carrier in a functional composition. This means that the mineral or mineral composite absorbs a particular substance to enable it to be administered in a particular form. For example, the mineral or mineral composition may prevent agglomeration or clumping of the functional ingredient (e.g. particular animal feed additive or fertilizer) and may thus make it easier to distribute. For example, the mineral or mineral composite may give the functional ingredient a more solid form and/or prevent the functional ingredient from being washed away from the composition/composite and thus improve the administration or distribution of the functional ingredient.

For example, the minerals and mineral composites disclosed herein may be used as a carrier in an animal feed composition. For example, the minerals and mineral composites disclosed herein may be used as a carrier in a fertilizer composition.

The minerals and mineral composites may, for example, be used as a carrier for one or more additives in animal feed, such as vitamins, organic acids, organic oils, choline chloride solutions, pigment dispersions, antioxidants, antibiotics, molasses, emulsifiers and lubricants.

The present invention thus further relates to the functional compositions disclosed herein, which comprise one or more of the minerals or mineral composites disclosed herein, together with a functional ingredient (e.g. particular animal feed additive or fertilizer). The minerals or mineral composites may be in accordance with any aspect or embodiment of the invention, including all combinations thereof.

Where the minerals and mineral composites disclosed herein are used as carriers, for example in animal feed compositions, the mineral or mineral composite may have an organic acid-absorption capacity of at least about 200 g/100 g of the mineral or mineral composition, for example at least about 250 g/100 g of the mineral or mineral composite, for example at least about 300 g/100 g of the mineral or mineral composite.

According to the present invention, the mineral disclosed herein is to be used in various personal care products, for example in deodorant compositions. For example, the minerals disclosed herein may be used to absorb sweat and/or odour. The use of the minerals disclosed herein in deodorant compositions may, for example, improve the drying time of the composition after it is applied to skin. The use of the minerals and mineral compositions disclosed herein in deodorant compositions may, for example, improve the skin feel of the composition, for example it may give a nude skin effect (skin feels as if there are no residues thereon) and/or it may not provoke or increase skin itching.

Where the minerals disclosed herein are used in personal care products (e.g. deodorant), the mineral or mineral has an organic acid-absorption capacity equal to or greater than about 220 g/100 g of the mineral or mineral composite (e.g. where the mineral is diatomaceous earth), for example at least about 250 g/100 g of the mineral or mineral composite, for example at least about 270 g/100 g of the mineral or mineral composite (e.g. where the mineral is perlite).

The present invention thus further relates to personal care compositions comprising diatomaceous earth.

## EXAMPLES

### Example 1

The organic acid- and water-absorption capacities of various minerals was determined by the method described above. The oil used was a commercially available blend of organic acids and surfactants, Fylax®. The results are shown in Table 1 below. The base flow energy (BFE) of some of these minerals was also determined by the method described above at 200 g of organic acid (Fylax®) per 100 g of mineral.

The synthetic silica used was Tixosil 38A® (Rhodia), having a particle size of about 60 $\mu$m. The diatomaceous earth used had a $d_{10}$ of 1 $\mu$m, a $d_{50}$ of 9 $\mu$m and a $d_{90}$ of 18 $\mu$m. The spray-died diatomaceous earth was made from the same diatomaceous earth that was tested. The spray-dried granules had a $d_{10}$ of 9 $\mu$m, a $d_{50}$ of 40 $\mu$m and a $d_{90}$ of 71 $\mu$m. The granulated diatomaceous earth was made from the same diatomaceous earth that was tested by adding 75 g of a 3.8 wt% PVOH solution to 100 g of the diatomaceous earth and then drying at 110°C overnight. The granulated diatomaceous earth had a $d_{10}$ of 4 $\mu$m, a $d_{50}$ of 15 $\mu$m and a $d_{90}$ of 43 $\mu$m. The expanded and un-milled perlite had a $d_{10}$ of about 100 $\mu$m, a $d_{50}$ of about 400 $\mu$m and a $d_{90}$ of about 900 $\mu$m. The expanded and classified perlite had a $d_{10}$ of about 20 $\mu$m, a $d_{50}$ of about 60 $\mu$m and a $d_{90}$ of about 130 $\mu$m. The expanded and milled perlite had a $d_{10}$ of 13 $\mu$m, a $d_{50}$ of 38 $\mu$m and a $d_{90}$ of 50 $\mu$m. The calcium silicate had a $d_{10}$ of about 6 $\mu$m, a $d_{50}$ of about 18 $\mu$m and a $d_{90}$ of about 40 $\mu$m. The median equivalent particle diameter ($d_{50}$ value) and other particle size properties referred to in Examples 1 to 3 are as measured by laser light particle size analysis using a CILAS (Compagnie Industrielle des Lasers) 1064 instrument. The (CILAS) measurements use a particle size measurement as determined by laser light particle size analysis using a CILAS (Compagnie Industrielle des Lasers) 1064 instrument. In this technique, the size of particles in powders, suspensions and emulsions may be measured using the diffraction of a laser beam, based on application of the Fraunhofer theory. The term $d_{50}$ (CILAS) used herein is the value determined in this way of the particle diameter at which there are 50% by volume of the particles which have a diameter less than the $d_{50}$ value. The CILAS 1064 instrument normally provides particle size data to two decimal places.

**Table 1.**

| Mineral | Organic acid-absorption capacity (g acid/100 g of mineral) | Water-absorption capacity (g acid/100 g of mineral) | BFE at 200 g organic acid per 100 g of mineral (mJ) |
|---|---|---|---|
| Synthetic Silica | 360 | 355 | 900 |
| Diatomaceous Earth | 300 | 280 | - |
| Spray-Dried Diatomaceous Earth (3% PVOH binder) | 380 | 355 | 910 |
| Granulated Diatomaceous Earth (with % PVOH) | 350 | 355 | - |
| Perlite (Expanded and un-milled) | 860 | 745 | 370 |

(continued)

| Mineral | Organic acid-absorption capacity (g acid/100 g of mineral) | Water-absorption capacity (g acid/100 g of mineral) | BFE at 200 g organic acid per 100 g of mineral (mJ) |
|---|---|---|---|
| Perlite (Expanded and classified) | 420 | 400 | 860 |
| Perlite (Expanded and milled) | 370 | 355 | - |
| Calcium Silicate (derived from diatomaceous earth) | 480 | 460 | - |

Example 2

The base flow energy of various minerals containing different levels of water or organic acid (commercially available blend of organic acids and surfactants, Fylax®) was determined by the method described above.

Commercial product 1 is the spray-dried synthetic silica Sipernat 2220®. Commercial product 2 is the granulated synthetic silica Tixosil 38A® (Rhodia). Commercial product 3 is the granulated synthetic silica Tixosil 38A®, pre-loaded with the organic acid Fylax®. The spray-dried diatomaceous earth is the mineral described in Example 1 above.

The results are shown in Figure 1. The x axis of Figure 1 shows the total amount of liquid (water or organic acid) in the composition. For example, 65 wt% of organic acid means that the composition comprises 65 g of acid for 100-65 = 35 g of mineral, which is equivalent to 185 g per 100 g of mineral. It was surprisingly found that the spray-dried diatomaceous earth remained free-flowing at relatively high water- and organic acid-loading levels. The performance was similar to that obtained using commercial product 3, which is known to be used in the animal feed industry.

In addition, a different diatomaceous earth having a $d_{10}$ of 0.6 $\mu$m, a $d_{50}$ of 8.5 $\mu$m and a $d_{90}$ of 18 $\mu$m, granulated with 3% polyvinyl alcohol binder, which was prepared in an Eirich mixer and then screened, was found to have a BFE of 1200 mJ when the mineral had a water content of 150 g/100 g of the mineral.

Example 3

Various minerals were mixed with synthetic sweat and left at 37°C for 24 hours. Olfactometric analyses were performed to determine odour concentration and to quantify odorant levels. A low odour concentration shows greater odour absorption. Sensory analyses were also conducted to provide information on the sensory properties of the sample products.

For each mineral product, a sample was prepared containing a mixture of 4g of mineral powder with 6.8 ml of synthetic sweat. The mixture was placed in a Nalophan® bag and after mixing, the closed bag was placed in an oven at 37°C for 24 hours before olfactometric analysis. In parallel, a bag containing only sweat was prepared in the same manner to serve as a reference.

Odour concentration refers to the persistence of an odour and its resistance to dilution. The higher the odour concentration value, the more difficult it is to dissipate the smell. The dilution factor is determined by a panel of six people. These individuals were selected by Odournet to detect defects in olfactory perception in compliance with EN 13725 standard.

Intensity measurements (based on German standard VDI 3882 part 1) were conducted with a panel of six people selected according to EN 13725 and in relation to a reference scale of n-butanol. During analyses, the individuals smell the product samples directly and assess their intensity on a scale of 0-5 (0 = imperceptible, 1 = very low, 2 = low, 3 = medium, 4 = high, 5 = very high). The relationship between intensity and odour concentration is not linear and is unique to each odorous gas mixture.

The talc had a $d_{10}$ of about 6 $\mu$m, a $d_{50}$ of about 26 $\mu$m and a $d_{90}$ of about 60 $\mu$m. The perlite had a $SiO_2$ content of 76.0 wt%, an $Al_2O_3$ content of 12.9 wt%, a $K_2O$ content of 4.6 wt%, a $Na_2O$ content of 3.5 wt% and a $d_{10}$ of about 8 $\mu$m, a $d_{50}$ of about 25 $\mu$m and a $d_{90}$ of about 50 $\mu$m. The diatomaceous earth had a $SiO_2$ content of 93.0 wt%, an $Al_2O_3$ content of 2.2 wt%, a $Fe_2O_3$ content of 1.2 wt% and a $d_{10}$ of about 3 $\mu$m, a $d_{50}$ of about 12 $\mu$m and a $d_{90}$ of about 45 $\mu$m. The particle size distribution of the samples was measured by laser diffraction (CILAS) as described above.

The results are shown in Table 2 below.

**Table 2.**

| Sample | Odour Concentration (UOE/m3) | Intensity |
|---|---|---|
| Synthetic sweat | 370 | 3.8 |

(continued)

| Sample | Odour Concentration (UOE/m3) | Intensity |
|---|---|---|
| Talc | 350 | 2.3 |
| Perlite | 270 | 1.8 |
| Diatomaceous Earth | 190 | 1.8 |

Exemplary deodorant compositions with and without the diatomaceous earth were prepared. It was found that the deodorant composition comprising the diatomaceous earth had a quicker drying time that the deodorant composition that did not comprise the diatomaceous earth. In addition, the deodorant composition comprising diatomaceous earth was found by testers to be easier to spread than the composition without the diatomaceous earth and have a better skin feel (no residues and no unpleasant feeling such as itching).

Example 4

A commercially available expanded perlite product having a $d_{50}$ of 20 $\mu$m, a specific gravity of 2.1 and a bulking value of 90, and a calcium silicate product derived from diatomaceous earth were used as the feed materials to make high absorption composite materials.

17.5 g of sodium silicate was dispersed in 70 g of DI water and then slowly added to 350 g of perlite mixture in a Hobart food mixer. The same amount of sodium silicate solution was used for all the perlite mixtures with different ratios. After mixing in for 15 minutes, the mixture was brushed through a 20 mesh screen with a 0.841 mm opening. The oversize particles were broken and forced through the screen by brushing. After drying in a 150°C oven overnight, the material was brushed through a 30 mesh (0.6 mm opening) screen.

The compositions prepared are shown in Table 3 below.

Table 3.

| Agglomerate | Perlite (g) | Calcium Silicate (g) | Sodium Silicate (g) | Water (g) |
|---|---|---|---|---|
| 1 | 87.5 | 262.5 | 17.5 | 70 |
| 2 | 175.0 | 175.0 | 17.5 | 70 |
| 3 | 262.5 | 87.5 | 17.5 | 70 |

The oil-absorption and water-absorption capacities of the perlite and calcium silicate minerals alone and the agglomerates were determined by the method described above using linseed oil. The bulk density of the samples was determined by measuring the weight of a given volume of the loosely packed sample. The wet density of the samples was also determined by placing a sample of known weight from about 1.00 to about 2.00 g in a calibrated 15 ml centrifuge tube to which deionized water is added to make up a volume of approximately 10 ml. The mixture is shaken thoroughly until all of the sample is wetted and no powder remains and additional deionized water is added around the top of the centrifuge tube to rinse down any mixture adhering to the side of the tube from shaking. The tube is centrifuged for 5 minutes at 2500 rpm on an IEC Centra® MP-4R centrifuge, equipped with a Model 221 swinging bucket rotor (International Equipment Company; Needham Heights, Massachusetts, USA). Following centrifugation, the tube is carefully removed without disturbing the solids, and the level (i.e., volume) of the settled matter is measured in $cm^3$. The centrifuged wet density of powder is readily calculated by dividing the sample weight by the measured volume. The particle size distribution of the samples was measured by laser diffraction using a Leeds & Northrup Microtrac Model X-100. The instrument is fully automated, and the results are obtained using a volume distribution formatted in geometric progression of 100 channels, generally running for 30 seconds with the filter on. The distribution is characterized using an algorithm to interpret data from the diffraction pattern which assumes the particles have spherical shape characterized by a diameter, d. For example, a median particle diameter is identified by the instrument as $d_{50}$, that is, 50% of the total particle volume is accounted for by particles having a diameter equal to or less than this value. The pore volume and pore diameter of the samples were also measured using a mercury porosimeter as described above. For bulk density and wet density, 1 lb/cf = 16.01846 $kg/m^3$.

The results are shown in Tables 4 and 5 below.

**Table 4.**

| Sample | $d_{10}$ ($\mu$m) | $d_{50}$ ($\mu$m) | $d_{90}$ ($\mu$m) | Pore Volume (mL/g) | Pore Diameter ($\mu$m) |
|---|---|---|---|---|---|
| Perlite | 9.34 | 21.26 | 46.80 | 4.87 | 3.23 |
| Calcium Silicate | 11.58 | 20.04 | 36.91 | 6.11 | 1.44 |
| Agglomerate 1 | 11.26 | 20.20 | 37.84 | 5.94 | 2.02 |
| Agglomerate 2 | 10.72 | 20.49 | 40.19 | 5.20 | 2.53 |
| Agglomerate 3 | 9.67 | 20.74 | 44.44 | 5.70 | 3.06 |

**Table 5.**

| Sample | Water-absorption capacity (%) | Oil-absorption capacity (%) | Bulk density (lb/cf) | Wet density (lb/cf) |
|---|---|---|---|---|
| Perlite | 198 | 179 | 5.1 | 16.3 |
| Calcium Silicate | 438 | 392 | 5.5 | 10.1 |
| Agglomerate 1 | 382 | 366 | 6.1 | 11.1 |
| Agglomerate 2 | 378 | 335 | 6.1 | 11.8 |
| Agglomerate 3 | 340 | 300 | 5.9 | 12.6 |

It was surprisingly found that the agglomerates comprising both perlite and calcium silicate minerals had a greater oil-absorption capacity and water-absorption capacity than the mean oil- and water-absorption capacities of the individual minerals.

Example 5

A diatomaceous earth product having a $d_{50}$ of 21.26 $\mu$m, $d_{90}$ of 46.8 $\mu$m, $d_{10}$ of 9.34 $\mu$m, specific gravity of 2.3 and bulking value of 19.2, and a calcium silicate product having a $d_{50}$ of 19.93 $\mu$m, $d_{90}$ of 36.54 $\mu$m, $d_{10}$ of 11.42 $\mu$m and specific gravity of 2.6 were used as the feed materials to make high absorption composite materials. The particle size properties were measured by laser analysis using a Microtrac instrument as described herein.

5 g of a sodium silicate binder was dispersed in 10 g of water and added to DE and calcium silicate in a food mixer. The same amount of binder was used for all the mixtures with different ratios. After mixing for 15 minutes, the mixture was brushed through a 20 mesh screen with a 0.841 mm opening. The oversize particles were broken and forced through the screen by brushing. After drying in a 150°C oven overnight, the material was brushed through a 30 mesh (0.6 mm) screen.

The compositions prepared are shown in Table 6 below.

**Table 6.**

| Composite | DE (g) | Calcium Silicate (g) | Binder (g) | Water (g) |
|---|---|---|---|---|
| 1 | 75.0 | 25.0 | 5 | 10 |
| 2 | 50.0 | 50.0 | 5 | 10 |
| 3 | 25.0 | 75.0 | 5 | 10 |

The oil-absorption and water-absorption capacities of the DE and calcium silicate minerals alone and the composites were determined by the method described above using linseed oil.

The wet density of the samples was also determined by placing a sample of known weight from about 1.00 to about 2.00 g in a calibrated 15 ml centrifuge tube to which deionized water is added to make up a volume of approximately 10 ml. The mixture is shaken thoroughly until all of the sample is wetted and no powder remains and additional deionized water is added around the top of the centrifuge tube to rinse down any mixture adhering to the side of the tube from shaking. The tube is centrifuged for 5 minutes at 2500 rpm on an IEC Centra® MP-4R centrifuge, equipped with a Model 221 swinging bucket rotor (International Equipment Company; Needham Heights, Massachusetts, USA). Following centrifugation, the tube is carefully removed without disturbing the solids, and the level (i.e., volume) of the settled matter is measured in cm³. The centrifuged wet density of powder is readily calculated by dividing the sample weight by the measured volume. For wet density, 1 lb/cf = 16.01846 kg/m³.

The surface area of the samples was determined according to the BET method by the quantity of nitrogen adsorbed on the surface of said particles so to as to form a monomolecular layer completely covering said surface (measurement according to the BET method, AFNOR standard X11-621 and 622 or ISO 9277). In certain embodiments, specific surface is determined in accordance with ISO 9277, or any method equivalent thereto.

Colour may be measured by any appropriate measurement technique now known to the skilled artisan or hereafter discovered. In one embodiment, the method for determining the colour of the products of this application uses Hunter scale L, a, b colour data collected on a Spectro/plus Spectrophotometer (Colour and Appearance Technology, Inc., Princeton, New Jersey). The L value indicates the level of light or dark, the a-value indicates the level of redness or greenness, and the b-value indicates the level of yellowness or blueness. BLB (blue light brightness) was calculated from the L, a and b value data. A krypton-filled incandescent lamp is used as the light source. The instrument is calibrated according to the manufacturer's instructions, generally using a highly polished black glass standard and a factory calibrated white opal glass standard. A plastic plate having a depression machined into it is filled with sample, which is then compressed with a smooth-faced plate using a circular pressing motion. The smooth-faced plate is carefully removed to insure an even, unmarred surface. The sample is then placed under the instrument's sample aperture for the measurements.

The results are shown in Table 7.

**Table 7.**

| Sample | Oil-absorption capacity (%) | Water-absorption capacity (%) | Wet density (lb/cf) | Surface area (m²/g) | L | a | b | BLB |
|---|---|---|---|---|---|---|---|---|
| DE | 167 | 211 | 16.3 | 2 | 94.75 | 0.09 | 1.82 | 87.31 |
| Calcium Silicate | 409 | 408 | 10.1 | 74 | 83.30 | 0.62 | 3.83 | 64.83 |
| Compos ite 1 | 233 | 374 | 11.1 | 18 | 89.92 | 0.53 | 3.58 | 76.26 |
| Compos ite 2 | 289 | 324 | 11.8 | 37 | 86.92 | 0.65 | 3.95 | 70.65 |
| Compos ite 3 | 350 | 274 | 12.6 | 52 | 84.64 | 0.66 | 3.96 | 66.85 |

It was surprisingly found that the composites had a greater oil-absorption capacity and water-absorption capacity than the oil- and water-absorption capacities of DE alone. In addition, the surface-area of the composites was higher than the surface-area of DE alone. Further, the colour of the composites was better than the colour of the calcium silicate alone.

## Claims

1. Use of a silica- or silicate-based mineral in a personal care product, wherein the silica or silicate mineral has an organic acid absorption capacity and/or an oil absorption capacity equal to or greater than about 220 g/100 g of the mineral according to the measurement method specified in the description, wherein the mineral is either naturally occurring or is made from a naturally occurring mineral, wherein the mineral is diatomaceous earth.

2. The use of a silica or silicate-based mineral of claim 1, which is in the form of a free-flowing granulate.

3. The use of a silica or silicate-based mineral of claim 1 or claim 2, having a base flow energy (BFE) equal to or less than about 1200 mJ, as measured using a Freeman Powder Rheometer model FT3 according to the measurement method specified in the description, at an organic acid content of 185 g/100 g of the mineral or at a water content of 150 g/100 g of the mineral.

4. The use of a silica or silicate-based mineral of any preceding claim, having a water absorption capacity equal to or greater than about 200 g/100 g of the mineral according to the measurement method specified in the description, for example equal to or greater than about 250 g/100 g of the mineral.

## Patentansprüche

1. Verwendung eines Minerals auf Siliciumdioxid- oder Silikatbasis in einem Körperpflegeprodukt, wobei das Siliciumdioxid- oder Silikatmineral ein Absorptionsvermögen für organische Säure und/oder ein Ölabsorptionsvermögen gleich oder größer als etwa 220 g/100 g des Minerals gemäß dem in der Beschreibung definierten Messverfahren hat, wobei das Mineral entweder natürlich vorkommend ist oder aus einem natürlich vorkommenden Mineral hergestellt ist, wobei das Mineral Diatomeenerde ist.

2. Verwendung eines Minerals auf Siliciumdioxid- oder Silikatbasis nach Anspruch 1, das in Form eines rieselfähigen Granulats vorliegt.

3. Verwendung eines Minerals auf Siliciumdioxid- oder Silikatbasis nach Anspruch 1 oder Anspruch 2, das eine Basisströmungsenergie (base flow energy - BFE) gleich oder kleiner als etwa 1200 mJ, gemessen unter Verwendung eines Freeman Powder Rheometer Modells FT3 gemäß dem in der Beschreibung definierten Messverfahren, bei einem Gehalt an organischer Säure von 185 g/100 g des Minerals oder bei einem Wassergehalt von 150 g/100 g des Minerals aufweist.

4. Verwendung eines Minerals auf Siliciumdioxid- oder Silikatbasis nach einem der vorhergehenden Ansprüche, das ein Wasserabsorptionsvermögen gleich oder größer als etwa 200 g/100 g des Minerals gemäß dem in der Beschreibung definierten Messverfahren aufweist, beispielsweise gleich oder größer als etwa 250 g/100 g des Minerals.

**Revendications**

1. Utilisation d'un minéral à base de silice ou de silicate dans un produit de soins personnels, dans laquelle le minéral à base de silice ou de silicate présente une capacité d'absorption d'acide organique et/ou une capacité d'absorption d'huile égale ou supérieure à environ 220 g/100 g du minéral selon le procédé de mesure spécifié dans la description, dans laquelle le minéral est soit d'origine naturelle, soit fabriqué à partir d'un minéral d'origine naturelle, dans laquelle le minéral est de la terre de diatomées.

2. Utilisation d'un minéral à base de silice ou de silicate selon la revendication 1, qui se présente sous la forme d'un granulé à écoulement libre.

3. Utilisation d'un minéral à base de silice ou de silicate selon la revendication 1 ou la revendication 2, présentant une énergie d'écoulement de base (BFE) égale ou inférieure à environ 1200 mJ, telle que mesurée à l'aide d'un rhéomètre à poudre Freeman modèle FT3 selon le procédé de mesure spécifié dans la description, à une teneur en acide organique de 185 g/100 g du minéral ou à une teneur en eau de 150 g/100 g du minéral.

4. Utilisation d'un minéral à base de silice ou de silicate selon une quelconque revendication précédente, présentant une capacité d'absorption d'eau égale ou supérieure à environ 200 g/100 g du minéral selon le procédé de mesure spécifié dans la description, par exemple égale ou supérieure à environ 250 g/100 g du minéral.

**Powder Flow as a Function of Organic Acid or Water Loading for Candidate Carrier Substrates**

FIG. 1

EP 3 374 079 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005005870 A1

- WO 2010042614 A

**Non-patent literature cited in the description**

- **J. BEAR**. The Equation of Motion of a Homogeneous Fluid: Derivations of Darcy's Law. *Dynamics of Fluids in Porous Media*, 1988, 161-177